# FASCICULE DE BREVET EUROPEEN

(11) **EP 1 333 816 B1**
(45) Date de publication et mention de la délivrance du brevet: **18.03.2009**
(21) Numéro de dépôt: 01996365.1
(22) Date de dépôt: 19.11.2001
(51) Int. Cl.: A61K 9/50, A61K 31/155, A61P 3/10

(54) **MEDICAMENT A BASE DE MICROCAPSULES D'ANTI-HYPERGLYCEMIANT A LIBERATION PROLONGEE ET SON PROCEDE DE PREPARATION**
MEDIKAMENT AUF DER BASIS VON EIN ANTIHYPERGLYKÄMIKUM ENTHALTENDEN MIKROKAPSELN MIT GESTEUERTER FREISETZUNG
MEDICINE BASED ON ANTI-HYPERGLYCAEMIC MICROCAPSULES WITH PROLONGED RELEASE AND METHOD FOR PREPARING SAME

(30) Priorité: 17.11.2000 FR 0014876
(43) Date de publication de la demande: 13.08.2003
(73) Titulaire: FLAMEL TECHNOLOGIES, 69693 Venissieux Cédex (FR)
(72) Inventeur: CASTAN, Catherine, 69530 ORLIENAS (FR); MEYRUEIX, Rémi, 69009 LYON (FR); SOULA, Gérard, F-69330 MEYZIEU (FR)
(74) Mandataire: Fleurance, Raphaël
(86) Numéro de dépôt international: PCT/FR2001/003625
(87) Numéro de publication internationale: WO 2002/039984

(56) Documents cités:
- EP-A- 0 502 642
- WO-A-00/28989
- US-A- 6 022 562

## Description

Le domaine de la présente invention est celui des formes galéniques orales permettant la libération prolongée de principes actifs anti-hyperglycémiants. Plus spécifiquement, la présente invention concerne un nouveau médicament administrable par voie orale et permettant la libération prolongée in vivo d'un biguanide tel que la metformine ou la buformine ou tout sel pharmaceutiquement acceptable de ces composés, comme, par exemple, le chlorhydrate de metformine.

Par formes galéniques orales à libération prolongée, on entend les formes galéniques orales permettant une libération ralentie des principes actifs, par rapport aux formes galéniques conventionnelles administrées selon la même voie. Cette définition est celle donnée par l*'Agence Européenne pour* l'Evaluation des Produits Médicaux, dans sa Note sur la Qualité *des Produits à Libération Modifiée* du 29 juillet 1999.

Cette définition exclut les formes galéniques orales à libération retardée, qui consistent en des formes galéniques permettant de retarder la libération des principes actifs, pendant une durée prédéfinie après administration, cette libération, équivalente à celle des formes galéniques conventionnelles, résultant alors d'un décalage de temps sans modification des autres paramètres pharmacocinétiques (Note sur la *Qualité des Produits à Libération Modifiée* du 29 juillet 1999, *Agence Européenne pour l'Evaluation des Produits Médicaux).*

L'art antérieur comprend de nombreuses propositions techniques pour obtenir des formes à libération prolongée de produits pharmaceutiques dans le but d'étendre la durée de la couverture thérapeutique et d'améliorer le confort des patients et le respect par ces derniers de la posologie. Mais très peu de travaux ont été consacrés à la mise au point de formes à libération contrôlée l'anti-hyperglycémiants.

Ces anti-hyperglycémiants, et en particulier le chlorhydrate de metformine, présentent une perméabilité intrinsèque faible dans les parties distales du tractus gastro-intestinal. Son absorption se fait donc essentiellement dans la partie supérieure du tractus gastro-intestinal. Sa biodisponibilité orale est de l'ordre de 40 à 60%. Elle diminue lorsque la dose augmente, ce qui suggère une absorption saturable ou limitée par la perméabilité et le temps de transit. Les produits présentant une absorption limitée à la partie supérieure du tractus gastro-intestinal, dits "à fenêtre d'absorption", sont considérés comme de mauvais candidats pour les formes orales à libération prolongée. L'administration de ces produits par un système conventionnel à libération prolongée peut effectivement se traduire par des concentrations plasmatiques inférieures au seuil thérapeutique et donc à un traitement inefficace.

Une autre caractéristique de ces anti-hyperglycémiants tels que le chlorhydrate de metformine est leur très grande solubilité dans l'eau : plus de 300 g/l à 25°C. Cela pose des problèmes lorsqu'il s'agit d'obtenir une formulation présentant une vitesse de libération faible et parfaitement contrôlée, sans décharge brutale " burst effect ". Pour pallier cela, il est généralement nécessaire d'utiliser de grandes quantités de polymères pour former une matrice ou une barrière capable de ralentir suffisamment la libération de la metformine pour obtenir le profil de concentration plasmatique souhaité et, dans ce cas, le formulateur a tout intérêt à privilégier les formes monolithiques qui offrent moins de surface à la diffusion du principe actif.

De plus, la dose de principe(s) actif(s) quotidienne peut être de l'ordre du gramme. C'est le cas notamment pour la metformine. Il en résulte que les formes à libération prolongée de metformine entre autres, peuvent présenter une grande taille. Une telle forme monolithique unitaire de grande taille peut subir une vidange gastrique aléatoire et donc rester durant un temps mal contrôlé en amont de sa fenêtre d'absorption. Il en résulte une absorption du principe actif aléatoire et mal contrôlée, en quantité et en durée. Ce genre de déconvenues ("bipasse") fréquentes a pour conséquence dans le cas des anti-hyperglycémiants, que la glycémie ne peut être correctement contrôlée, ce qui peut avoir des conséquences extrêmement préjudiciables pour le patient diabétique.
La forme galénique monolithique de grande taille peut également se trouver bloquée dans les méandres du tractus gastro-intestinal. Il se produit alors une libération massive et très localisée du principe actif ("dose dumping")qui, non seulement ne sera pas absorbé selon le profil recherché, mais qui de surcroît est susceptible de provoquer des lésions locales des tissus à l'endroit de la libération massive.

Ainsi, l'art antérieur ne décrit que des formes monolithiques capables de demeurer un certain temps dans l'estomac (gastrorétention), de façon à libérer la metformine en amont de sa fenêtre d'absorption.

La demande de brevet WO 98/55107 décrit des comprimés comportant une matrice de polymère hydrophile de haut poids moléculaire (polyoxyéthylène) contenant la metformine. Une fois ingérée, cette matrice gonfle jusqu'à atteindre des grandes dimensions (e.g.7,2 mm diamètre x 8,8 mm longueur)favorisant sa rétention par l'estomac, tout en limitant la vitesse de dissolution de la metformine.

Le brevet WO 99/47125 décrit un comprimé monolithique à libération contrôlée formé :
- d'un coeur contenant :
   - l'actif anti-hyperglycémiant (metformine),
   - un agent liant hydrosoluble (polyvinylpyrrolidone),
   - un promoteur d'absorption (sel biliaire),
- et d'une membrane semi-perméable (dérivé insoluble de cellulose) enrobant le coeur et percée d'au moins un trou.
De manière traditionnelle dans les techniques de fabrication de comprimés, les comprimés selon le WO 99/47125 sont obtenus à partir de granulés non enrobés, préparés par granulation humide, c'est à dire agglomération de particules de metformine à l'aide de l'agent liant susmentionné. Les granulés ont une taille significativement supérieure à celle des particules de metformine de départ.
Cette forme galénique est censée avoir une couverture thérapeutique sur 24 heures après administration orale à l'état nourri.
L'un des inconvénients de cette forme galénique réside dans la présence de ce promoteur d'absorption qui peut fragiliser la paroi intestinale et peut, en administration prolongée, avoir des effets secondaires indésirables.
Un autre inconvénient est que cette forme "comprimé" présente un temps de résidence gastrique variable, à la différence d'une forme galénique microparticulaire, dont le temps de résidence est pondéré par le grand nombre de particules.

Le brevet WO 99/47128 décrit un système galénique oral à libération prolongée, qui permet une résidence prolongée dans l'estomac.
Il s'agit d'une forme adaptée à des principes actifs dotés d'une forte solubilité dans l'eau et présentant une fenêtre d'absorption limitée à la partie haute du tractus gastro-intestinal (Metformine). Ce système est biphasique et comprend:
➢ une phrase interne particulaire formée de granules individualisés chargés en PA (principe(s) actif(s)). La particularité de ces granules est d'être non enrobés et de comporter un ou plusieurs excipients qui peuvent être :
   o un polymère hydrophobe : copolymère de l'acide (méth)acrylique (EUDRAGIT^{®}), éthylcellulose,
   o et/ou un polymère hydrophile : carboxyméthylcellulose de sodium ou alginate de sodium,
   o et/ou d'autres composés hydrophobes : cires, alcools gras, esters d'acide gras,
➢ et une phase continue solide externe dans laquelle sont noyées les particules de la phase interne, cette phase solide externe continue comportant :
   o un ou plusieurs polymères hydrophiles : [hydroxypropylméthyl-cellulose -HPMC- (de viscosité 5 cps et 1.10⁵ cps), cellulose microcristalline],
   o et/ou un ou plusieurs polymères hydrophobes,
   o et/ou un ou plusieurs autres composés hydrophobes (cires, alcools gras, esters d'acide gras).
Ce système galénique est, de préférence, sous forme de comprimés oblongs. Il est présenté comme ayant un temps de résidence accru dans la partie haute du tractus gastro-intestinal (estomac/intestin grêle) par effet d'augmentation de taille, sans toutefois atteindre une limite supérieure conduisant à l'occlusion.
Un inconvénient de cette forme galénique est qu'elle présente un temps de résidence gastrique variable, à la différence d'une forme galénique microparticulaire, dont le temps de résidence est pondéré par le grand nombre de particules. Par ailleurs, il est vraisemblable que ce système galénique selon le WO 99/47128 (de préférence un comprimé) ait une faible tenue mécanique en milieu gastrique. Dans une telle hypothèse la libération du PA ne serait plus contrôlée.

Ces trois inventions se référent à des formes monolithiques de dimensions importantes, devant être ingérées comme telles. Ainsi :
o pour une dose de 1 g de metformine: le brevet WO 98/55107 propose 8 comprimés de 10,4 x 6,6 mm contenus dans 4 gélules ;
o pour une dose de 1 g de metformine: le brevet WO 99/47128 propose 2 comprimés oblongs de grandes dimensions ;
o pour une dose de 850 mg de metformine: le brevet

WO-99/47125 propose un comprimé de diamètre 12 mm.
Ces formes peuvent poser des problèmes d'observance pour les patients ayant des difficultés à avaler.
De plus, le profil de concentration plasmatique obtenu à partir de ces systèmes est fortement conditionné par le temps de résidence dans l'estomac, lequel peut faire l'objet de grandes variations interindividuelles. Les systèmes monolithiques subissent et quelquefois amplifient l'effet de ces variations interindividuelles, ce qui peut conduire à l'inefficacité du traitement chez une portion non négligeable de la population traitée.
Enfin, ces systèmes galéniques sont susceptibles d'entraîner soit des problèmes de bipasse de la fenêtre d'absorption, soit des problèmes d'accumulation localisée du principe actif, et de lésions subséquentes.

La demande PCT WO-96/11675 décrit des microcapsules médicamenteuses et/où nutritionnelles pour l'administration per os de principe(s) actif(s), à l'exclusion de l'aspirine et sans précision d'une classe particulière de principes actifs, à savoir les anti-hyperglycémiants et en particulier la metformine. Ces micro-capsules médicamenteuses sont constituées par des particules de principe(s) actif(s) (sans que les anti-hyperglycémiants soient spécifiés), recouvertes chacune d'une pellicule d'enrobage comprenant au moins un polymère filmogène P1, au moins un polymère azoté P2, au moins un plastifiant et au moins un tensioactif et/ou lubrifiant. Les micro-capsules médicamenteuses selon le WO-96/11675 n'apportent pas de solution au problème particulier de la couverture thérapeutique sur 24 heures par des anti-hyperglycémiants à fenêtre d'absorption dans les parties hautes du tractus gastro-intestinal, très solubles dans l'eau et devant être ingérées à hautes doses pour chaque prise (1 g par jour).

Le document WO-00/28989 décrit des compositions à libération retard se présentant sous la forme de gélules contenant de multiples coeurs de granulés comprenant un agent de sensibilisation à l'insuline et un autre agent antidiabétique, qui peut être un biguanide tel que la metformine. Les coeurs de granulés peuvent être enrobés d'une composition entérique et notamment constituée par un polymère filmogène tel que l'Eudragit^{®} L100-55. Toutefois, ce document ne décrit pas un médicament anti-hyperglycémiant administrable *per os*, se présentant sous la forme de microparticules permettant la libération prolongée du ou des principe(s) actif(s) anti-hyperglycémiant(s).

Dans un tel état de la technique, l'un des objectifs essentiels de la présente invention est de fournir un nouveau système galénique, pour l'administration orale de principe(s) actif(s) anti-hyperglycémiant(s), ce système devant permettre d'obtenir une couverture thérapeutique performante sur 24 heures en surmontant les problèmes de bipasse de la fenêtre d'absorption et de libération massive localisée de principe(s) actif(s).

Un objectif de la présente invention est de proposer une forme pharmaceutique constituée d'un grand nombre, de l'ordre de plusieurs milliers, de microcapsules d'anti-hyperglycémiant(s), et particulièrement de metformine, cette multiplicité de microcapsules assurant statistiquement une bonne reproductibilité de la cinétique de transit de l'anti-hyperglycémiant (metformine), dans tout le tractus gastro-intestinal. Il en résulte un meilleur contrôle de la biodisponibilité, et donc pour le patient un risque moindre d'hyperglycémie ou d'hypoglycémie.

Un objectif de la présente invention est de proposer une forme galénique multi-microcapsulaire à libération prolongée d'anti-hyperglycémiant(s), et particulièrement de metformine, cette forme galénique étant constituée par un comprimé dispersible dans un liquide ou dans la bouche, par un comprimé effervescent, ou par de la poudre en sachets.

Un autre objectif de la présente invention est de proposer une forme multi-microcapsulaire à libération prolongée d'anti-hyperglycémiant(s), et particulièrement de metformine, qui conduise, après administration orale, à un pic plasmatique après plus de 6 heures approximativement.

Un autre objectif de la présente invention est de proposer une forme multi-microcapsulaire à libération prolongée d'anti-hyperglycémiant(s), et particulièrement de metformine, dont la biodisponibilité n'est pas réduite par une administration à l'état nourri.

Un autre objectif de la présente invention est de proposer une forme multi-microcapsulaire à libération prolongée d'anti-hyperglycémiant(s), et particulièrement de metformine, assurant une couverture thérapeutique suffisante pour une administration une ou deux fois par jour du principe actif.

Un autre objet de la présente invention est d'obtenir un système multi-microcapsulaire assurant la libération in vitro des produits anti-hyperglycémiants sur plus de 8 heures tout en évitant l'emploi de fortes quantités de polymères, le titre en principe(s) actif(s) demeurant comparable voire supérieur à celui de formes monolithiques.

Un autre objet de la présente invention est de fournir un procédé simple et économique de préparation de la forme galénique multi-microcapsulaire sus-évoquée.

Les objectifs exposés ci-dessus, parmi d'autres, sont obtenus par l'invention qui propose en premier lieu un médicament à base d'au moins un anti-hyperglycémiant choisi parmi les biguanides, administrable par voie orale caractérisé:
■ en ce qu'il comprend une pluralité de microcapsules constituées chacune par un coeur comportant au moins un anti-hyperglycémiant et par une pellicule d'enrobage appliquée sur le coeur et permettant la libération prolongée in vivo de (des) l'anti-hyperglycémiant(s),
   *à l'exclusion des pellicules d'enrobage constituées par des compositions entériques et des pellicules d'enrobage de composition ci-après:*
   1 - *au moins un polymère filmogène (P1) insoluble dans les liquides du tractus, présent à raison de 50 à 90 %, préférence 50 à 80 % en poids sur sec par rapport à la masse totale de la composition d'enrobage et constitué par au moins un dérivé non hydrosoluble de la cellulose, à savoir l'éthylcellulose ét*/*ou l'acétate de cellulose ;*
   2 - *au moins un polymère azoté (P2) présent à raison de 2 à 25 %, de préférence 5 à 15 % en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone ;*
   3 - *au moins un plastifiant présent à raison de 2 à 20 %, de préférence de 4 à 15 % en poids sur sec par rapport à la masse totale de la composition d'enrobage et constitué par au moins l'un des composés suivants les esters du glycérol, les phtalates, les citrates, les sébacates, les esters de l'alcool cétylique, l'huile de ricin, l'acide salicylique et la cutine ;*
   4 - *et éventuellement au moins un agent tensioactif et*/*ou lubrifiant, présent à raison de 2 à 20 %, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et*/*ou oléique étant préférés, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants comme les stéarates de calcium, de magnésium, d'aluminium ou de zinc, ou comme le stéarylfumarate de sodium et*/*ou le béhénate de glycérol; ledit agent pouvant comprendre un seul ou un mélange des susdits produits ;*
■ en ce que ces micro-capsules possèdent une granulométrie comprise entre 50 et 1 000 microns, de préférence entre 100 et 750 microns, et, plus préférentiellement encore, entre 200 et 500 microns.

Par compositions entériques, on entend les compositions qui confèrent à l'enrobage une résistance à pH acide (pH gastrique) et qui permettent une libération du ou des principe(s) actif(s) lors d'une remontée de pH.

Ainsi, le médicament selon l'invention est particulièrement adapté aux principes actifs anti-hyperglycémiants choisis parmi les biguanides, qui ont pour caractéristique d'avoir une fenêtre d'absorption située dans les parties hautes du tractus gastro-intestinal (estomac et début intestin grêle), qui sont très solubles dans l'eau et dont la posologie est de l'ordre de 1g par jour, ce qui impose l'ingestion de grandes masses de produit par prise.

Ce médicament sous une forme galénique "multimicrocapsulaire" composée d'une pluralité de microcapsules, limite nécessairement pour des raisons statistiques le risque de bipasse de la fenêtre d'absorption, et supprime le risque d'accumulation localisée de principe(s) actif(s). Il en résulte une absorption optimale d'anti-hyperglycémiants dans la fenêtre d'absorption, en quantité et sur une durée telles que la couverture thérapeutique peut être garantie sur au moins 12h avec toute la sécurité thérapeutique souhaitable(contrôle de la glycémie). En effet, le grand nombre de particules (e.g. de l'ordre de 10 000) permet une distribution reproductible, diminuant ainsi les risques d'hyperglycémie et d'hypoglycémie.

Les anti-hyperglycémiants choisis parmi les biguanides plus particulièrement concernés par l'invention sont ceux choisis dans le groupe comprenant la metformine et ses sels, tels que le chlorhydrate de metformine.

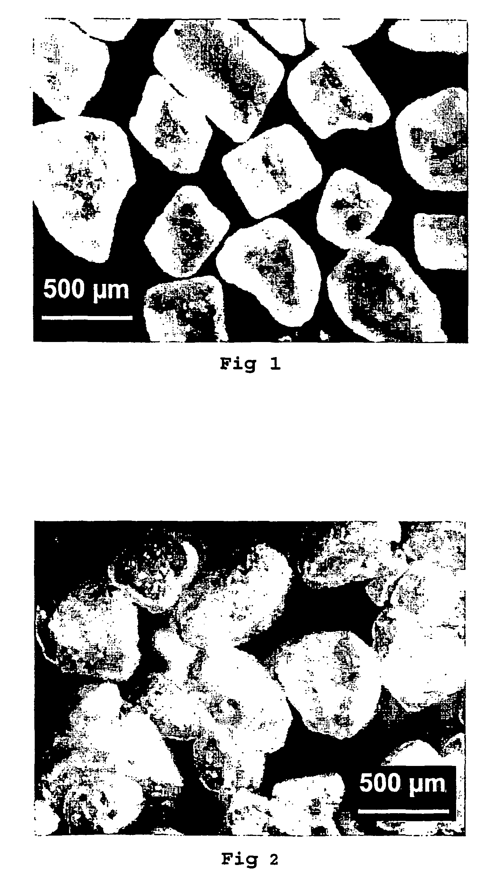

Les figures 1 et 2 annexées sont des photographies (respectivement avant et après un test de dissolution, cf exemples infra) de plusieurs microcapsules constitutives du médicament selon l'invention. Ces photos montrent bien que chaque microcapsule individualisée comprend un coeur enveloppé dans une pellicule d'enrobage gouvernant la libération prolongée du ou des principe(s) actif(s) anti-hyperglycémiants.

La taille de chaque micro-capsule est inférieure à 1 mm et en pratique comprise entre 200 et 500 µm, comme cela apparaît sur la photo de la figure 1. Il doit être souligné qu'il ne s'agit pas d'un agglomérat de particules d'anti-hyperglycémiants en granules de taille supérieure à 1 mm et dont la matrice est formée par un liant polymère.

Le médicament selon l'invention concerne des formes pulvérulentes sèches ou en suspension dans un liquide ou bien encore des formes délitées dans la bouche ou dans un liquide.

En fait, le médicament selon l'invention peut être assimilé à un système galénique original "multi-microcapsulaire" destiné à être facilement administrable *per os* et permettant une libération prolongée in vivo, garantissant une couverture thérapeutique sur au moins 12 heures et, de préférence, sur au moins 24 heures.

De préférence, la taille et l'enrobage des microcapsules sont choisis de telle sorte que, toutes choses égales par ailleurs, sa biodisponibilité lors d'une administration orale à l'état nourri est au moins égale à sa biodisponibilité lors d'une administration orale à jeun.

Suivant une caractéristique préférée de l'invention, la pellicule d'enrobage des microcapsules est conçue de telle sorte qu'après ingestion d'une dose donnée d'anti-hyperglycémiant, le temps (Tmax) correspondant à la concentration plasmatique maximale dans la courbe de la concentration plasmatique en fonction du temps, soit supérieur ou égal à 6 heures et la biodisponibilité donnée par l'aire sous la courbe (AUC) est supérieure ou égale à 60 %, de préférence à 80 % par rapport à celle obtenue avec la même dose d'anti-hyperglycémiant à libération immédiate. Ce type de courbes donnant la concentration plasmatique en fonction du temps écoulé après l'ingestion, rend compte de la couverture et de l'efficacité thérapeutique du médicament. Il y a lieu de constater qu'elles sont tout à fait satisfaisantes pour le médicament multi-microcapsulaire et anti-hyperglycémiant selon l'invention.

Ainsi, le médicament selon l'invention ouvre des perspectives tout à fait intéressantes pour le traitement des diabètes de type II, seul ou en combinaison avec d'autres médicaments anti-diabétiques tels que l'insuline.

De manière tout à fait surprenante et inattendue, le système galénique oral multi-microcapsulaire selon l'invention ne requiert pas la mise en oeuvre d'importantes quantités de polymères excipients relativement à la masse d'anti-hyperglycémiants. Contrairement à ce qui existe pour les systèmes galéniques connus de type monolithique de grandes dimensions.

Ainsi, suivant une disposition avantageuse de l'invention, la fraction massique moyenne en anti-hyperglycémiant des microcapsules est supérieure à 50%, de préférence supérieure ou égale à 60%.

Les problèmes techniques auxquels l'invention apporte remède, sont plus spécifiquement ceux rencontrés pour les anti-hyperglycémiants et en particulier ceux choisis parmi les biguanides de préférence dans le groupe de biguanides comprenant la metformine et la buformine et leurs sels, la metformine et ses sels étant particulièrement préférés.

Lé médicament selon l'invention peut également être défini par des caractéristiques de libération in vitro du ou des anti-hyperglycémiants(s), par dissolution en milieu aqueux de la pellicule d'enrobage. D'où il s'ensuit que dans un test de dissolution in vitro dénommé dissolutest de type II conformément à la Pharmacopée, la dissolution de l'anti-hyperglycémiant s'étend sur au moins 8 heures, de préférence au moins 20 heures.

Le médicament multi-microcapsulaire selon l'invention peut se présenter sous diverses formes galéniques, dont notamment :
- comprimés délitables dans la bouche,
- comprimés délitables par effervescence dans un liquide (eau),
- comprimés délitables dans un liquide (eau),
- poudres conditionnées en sachet de doses données,
- suspensions de microcapsules dans un liquide (eau),
- gélules contenant une poudre de microcapsules.

Suivant un mode de réalisation particulier mais non limitatif de l'invention, le médicament multi-microcapsulaire est constitué par une forme galénique dont la dose d'anti-hyperglycémiant est comprise entre 800 et 1200 mg, de préférence entre 900 et 1100 mg, et plus préférentiellement encore de l'ordre de 1000 mg. Une telle dose convient particulièrement pour le traitement des diabètes de type II, selon une posologie performante permettant de contribuer à maintenir la glycémie à des taux acceptables 24 heures après la prise.

Avantageusement, ce médicament multi-microcapsulaire, dont la dose d'anti-hyperglycémiant est comprise entre 800 et 1200 mg, de préférence entre 900 et 1100 mg, et plus préférentiellement encore de l'ordre de 1000 mg, se compose de plusieurs milliers de microcapsules telles que définies ci-dessus, cette multiplicité assurant une bonne reproductibilité du transit gastro-intestinal de l'anti-hyperglycémiant, réduisant ainsi le risque d'hypoglycémie ou d'hyperglycémie pour le patient.

Pour détailler quelque peu la structure des microcapsules, on précise que le coeur desdites microcapsules peut être par exemple un granulé comportant de l'anti-hyperglycémiant et des excipients de granulation et/ou une particule d'anti-hyperglycémiant, de préférence un monocristal.

Dans le coeur des microcapsules, l'anti-hyperglycémiant peut être associé à un ou plusieurs excipients. C'est notamment le cas lorsque le coeur est constitué par un granulé. Les excipients alors mis en oeuvre sont ceux qui sont traditionnels dans la granulation.
En pratique, le pelliculage déposé sur chaque granulé peut être constitué par une ou plusieurs macromolécule(s) filmogène(s) bien connue(s) de l'homme de l'art pour réaliser les formes à libération prolongée. Par exemple, et sans que la liste soit exhaustive, il peut être choisi parmi les familles suivantes : éthers de cellulose, éthers / esters de cellulose, esters de cellulose, diesters de cellulose, triesters de cellulose, acylate de cellulose, diacylate de cellulose, triacylate de cellulose, diacétate et triacétate de cellulose, acétate propionate de cellulose, acétate butyrate de cellulose, poly-méthacrylates, cires, copolymères d'acétate de vinyle.
De préférence, la macromolécule filmogène est l'éthylcellulose, l'Eudragit® RS, l'Eudragit® RL, l'acétate de cellulose.

Encore plus préférentiellement, on utilisera les associations de dérivés cellulosiques et d'au moins un polymère hydrophile pharmaceutiquement acceptable.

La fraction massique en dérivés cellulosiques est avantageusement comprise entre 30 et 90% et plus avantageusement encore entre 50 et 80%.

Le pelliculage peut aussi contenir les excipients communément utilisés comme plastifiants. Ils peuvent être choisis parmi la liste non exhaustive suivante : acétyl tributyl citrate, acétyl triéthyl citrate, glycérides acétylés, huile de ricin, dibutylphtalate, diéthylphtalate, diéthylsébacate, dibutylsébacate, diméthylphtalate, glycérol, glycérol monostéarate, glycéryl triacétate, polyéthylène glycol, copolymères polyoxyéthylène/polyoxypropylène, propylène glycol, tributyl citrate, triéthylcitrate, adipate, azélate, benzoate, citrate, esters d'acide citrique, huiles végétales, sorbitol, diéthyloxalate, diéthylmalate, diéthylfumarate, dibutylsuccinate, diéthylmalonate, dioctylphtalate, glycéroltributyrate,
D'autres excipients d'usage courant peuvent être introduits dans l'enrobage comme les charges solubles ou insolubles (talc, sels minéraux, sucres, polyvinylpyrrolidone, polyethylène glycol...), les lubrifiants, les colorants ou les pigments.

Selon un autre de ses aspects, l'invention vise le procédé de préparation du médicament tel que défini ci-dessus.

Ce procédé consiste :
❖ à mettre en oeuvre des granulés comportant de (des) l'anti-hyperglycémiant(s) choisi(s) parmi les biguanides et des excipients de granulation ou bien de particules d'anti-hyperglycémiant(s) sensiblement pur(s), de préférence des monocristaux d'anti-hyperglycémiant(s) ;
❖ puis à pulvériser sur ces granulés et/ou ces particules, une solution d'enrobage comprenant un ou plusieurs produits sélectionnés dans le groupe comprenant:
   - les macromolécules filmogènes, de préférence choisies dans le groupe comprenant : éthers de cellulose, éthers/esters de cellulose, esters de cellulose, diesters de cellulose, triesters de cellulose, acylate de cellulose, diacylate de cellulose, triacylate de cellulose, diacétate et triacétate de cellulose, acétate propionate de cellulose, acétate butyrate de cellulose, poly-méthacrylates, cires, copolymères d'acétate de vinyle ;
      l'éthylcellulose, l'Eudragit® RS, l'Eudragit® RL, l'acétate de cellulose étant particulièrement préférés;
   - les plastifiants, de préférence choisis parmi la liste non exhaustive suivante : acétyl tributyl citrate, acétyl triéthyl citrate, glycérides acétylés, huile de ricin, dibutylphtalate, diéthylphtalate, diéthylsébacate, dibutylsébacate, diméthylphtalate, glycérol, glycérol monostéarate, glycéryl triacétate, polyéthylène glycol, copolymères polyoxyéthylène/polyoxypropylène, propylène glycol, tributyl citrate, triéthylcitrate, adipate, azélate, benzoate, citrate, esters d'acide citrique, huiles végétales, sorbitol, diéthyloxalate, diéthylmalate, diéthylfumarate, dibutylsuccinate, diéthylmalonate, dioctylphtalate, glycéroltributyrate;
   - et éventuellement d'autres excipients sélectionnés parmi les charges solubles ou insolubles (talc, sels minéraux, sucres, polyvinylpyrrolidone, polyéthylène glycol...), les lubrifiants, les colorants ou les pigments ;
en utilisant une technologie prévue à cet effet et connue de l'homme de l'art, telle que celle faisant intervenir un système WURSTER® de la société GLATT ou un système PRECISIOCOATER® de la société AEROMATIC.

Comme déjà signalé ci-dessus, les granulés susceptibles de former le coeur des microcapsules sont obtenues par des techniques conventionnelles de granulation.
Les excipients de granulation mis en oeuvre sont bien connus de l'homme de l'art et sont notamment ceux exemplifiés ci-dessus.

Les caractéristiques innovantes du procédé résultent des matériaux mis en oeuvre et de la combinaison des paramètres physiques judicieusement sélectionnés.

Conformément à l'invention, il est également proposé à titre de solution aux problèmes évoqués au début du présent exposé, à savoir : bipasse de la fenêtre d'absorption, libération massive et localisée de l'anti-hyperglycémiant choisis parmi les biguanides, forme galénique avalable aisément, le tout dans une perspective de couverture thérapeutique efficace et sûre pendant au moins 12h (contrôle de la glycémie),
d'utiliser une pluralité de microcapsules:
◆ constituées chacune par un coeur comportant au moins un anti-hyperglycémiant choisi parmi les biguanides et par une pellicule d'enrobage appliquée sur le coeur et permettant la libération prolongée in vivo de (des) l'anti-hyperglycémiant(s),
◆ et possédant une granulométrie comprise entre 50 et 1 000 microns, de préférence entre 100 et 750 microns, et plus préférentiellement encore, entre 200 et 500 microns ;
pour fabriquer un médicament à base d'au moins un anti-hyperglycémiant choisi parmi les biguanides et administrable par voie orale, avalable aisément et qui contribue au contrôle de la glycémie (réduction du risque d'hypoglycémie ou d'hyperglycémie) sur au moins 12h, de manière sûre, en limitant les problèmes de bipasse, d'accumulation et de libération localisée et massive d'anti-hyperglycémiant(s).

Selon encore un autre de ses objets, la présente invention concerne une méthode de traitement des diabètes de type II, pour lesquels on a recours à un médicament tel que défini ci-dessus en tant que produit *per se* ou en tant que produit obtenu par le procédé sus-décrit.

Les exemples qui suivent permettront de mieux comprendre l'invention et d'appréhender tous ces avantages et toutes ses variantes de réalisation.

### EXEMPLES

### Description des Figures :

- Les figures 1 et 2 sont des photographies de microcapsules respectivement avant et après le test de dissolution dissolutest mis en oeuvre et défini dans les exemples.
- La figure 3 est une courbe donnant le pourcentage de dissolution (dissolutest) en fonction du temps, de microcapsules de metformine selon l'exemple 1.
- La figure 4 est une courbe donnant le pourcentage de dissolution (dissolutest) en fonction du temps, de microcapsules de metformine selon l'exemple 2.

### EXEMPLE 1 :

159,5 g d'acide stéarique et 159,5 g d'éthylcellulose sont dissous dans 2870 g d'isopropanol maintenu à 50°C. Cette solution est pulvérisée sur 700 g de cristaux de metformine, HCl de diamètre moyen compris entre 100 et 200 µm et chargés dans un Spray coater Glatt GPCG1. Les conditions de pelliculage sont : température produit : 38-42°C, vitesse de pulvérisation : 10 g/min, pression d'atomisation : 2 bars.
Les microcapsules obtenues ont été testées dans un dissolutest de type II conforme à la Pharmacopée dans un milieu tampon KH₂PO₄ / NaOH à pH 6,8, maintenu à 37°C et agité à 10 tours/min.

Il apparaît que les microcapsules n'ont pas été modifiées extérieurement par le test de dissolution. Cela prouve qu'elles comportent bien un enrobage au travers duquel la metformine a diffusé lors du test de dissolution et qui n'a pas été affecté par la dissolution.

Le profil de dissolution obtenu est le suivant :

**TABLEAU 1**

| Temps | Metformine dissoute |
|---|---|
| (heure) | (%) |
| 2 | 14,7 |
| 4 | 34,4 |
| 8 | 69,4 |
| 12 | 87,5 |
| 16 | 94,3 |
| 20 | 97,1 |

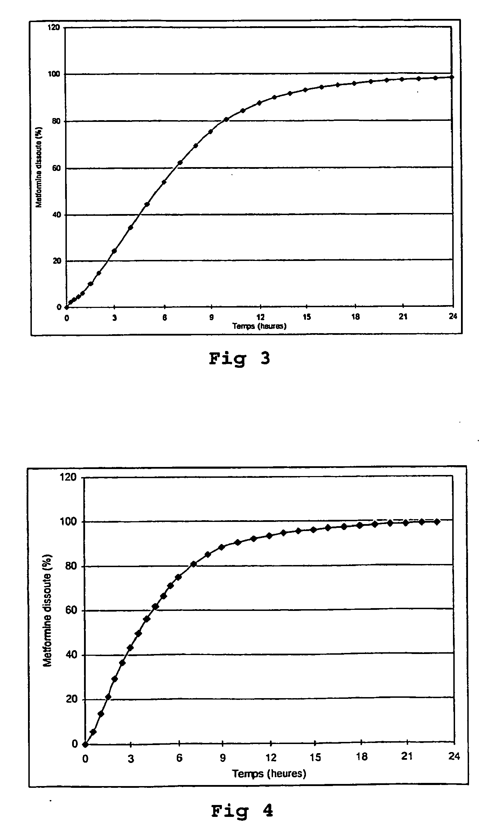

Le profil de dissolution du produit préparé dans cet exemple est représenté figure 3.

### EXEMPLE 2 :

51,13 g d'éthylcellulose et 5,73 g d'huile de ricin sont dissous dans un mélange de 393 g d'acétone et 262 g d'isopropanol. Cette solution est pulvérisée sur 200 g de cristaux de metformine, HCl de diamètre moyen compris entre 200 et 500 µm et chargés dans un Spray coater Niro CC1. Les conditions de pelliculage sont : température produit : 38-42°C, vitesse de pulvérisation : 4 g/min, pression d'atomisation : 1 bar.
Les microcapsules obtenues ont été testées dans un dissolutest de type II conforme à la Pharmacopée dans un milieu tampon KH₂PO₄ / NaOH à pH 6,8, maintenu à 37°C et agité à 10 tours/min. Le profil de dissolution obtenu est le suivant :

**TABLEAU 2**

| Temps | Metformine dissoute |
|---|---|
| (heure) | (%) |
| 2 | 29,4 |
| 4 | 56,0 |
| 8 | 85,2 |
| 12 | 93,6 |
| 16 | 96,9 |
| 20 | 98,7 |

Le profil de dissolution du produit préparé dans cet exemple est représenté figure 4.

### Exemple 3 :

Deux gélules de taille 00 contenant chacune 500 mg de metformine enrobée dans les microcapsules décrites dans l'exemple 2, ou bien 4 comprimés Glucophage contenant chacun 250 mg de metformine sont administrés à 12 sujets sains après dîner avec 250 ml.
Des échantillons de sang sont prélevés à 0, 0,5, 1, 2, 3, 4, 5, 6, 8, 10, 12, 16, 20, 24, 36 heures après administration pour analyse de la concentration en Metformine.
Le profil de concentration plasmatique moyen met en évidence une augmentation du temps correspondant au maximum de concentration plasmatique, et ceci sans diminution très notable de la bio-disponibilité évaluée par l'aire sous le profil de concentration plasmatique entre les instants et 36 heures.
Les principaux paramètres pharmacocinétiques sont reportés dans le tableau 3 ci-dessous.

**TABLEAU 3**

| | Tmax (h) | Cmax | AUC 0-36 h | Biodisponibilité |
|---|---|---|---|---|
| | | (ng/ml) | (ng.h/ml) | relative (%) |
| Glucophage | 3,5 | 1280 | 10500 | 100 |
| Micro capsules de l'exemple 2 | 7,1 | 1015 | 9660 | 92 |

Les microcapsules de l'invention représentent donc une avancée conséquente dans le domaine de l'administration de la metformine par voie orale pour le traitement du diabète.

## Revendications

1. Médicament, à base d'au moins un anti-hyperglycémiant administrable par voie orale, choisi parmi les biguanides, de préférence dans le groupe de biguanides comprenant la metformine et la buformine et leurs sels, la metformine et ses sels étant particulièrement préférés,
**caractérisé :**
■ **en ce qu'**il comprend une pluralité de microcapsules constituées chacune par un coeur comportant au moins un anti-hyperglycémiant et par une pellicule d'enrobage appliquée sur le coeur et permettant la libération prolongée in vivo de (des) l'anti-hyperglycémiant(s),
à l'exclusion des pellicules d'enrobage constituées par des compositions entériques et des pellicules d'enrobage de composition ci-après :
1 - au moins un polymère filmogène (P1) insoluble dans les liquides du tractus, présent à raison de 50 à 90 %, de préférence 50 à 80 % en poids sur sec par rapport à la masse totale de la composition d'enrobage et constitué par au moins un dérivé non hydrosoluble de la cellulose, à savoir l'éthylcellulose et/ou l'acétate de cellulose ;
2 - au moins un polymère azoté (P2) présent à raison de 2 à 25 %, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, à savoir le polyacrylamide et/ou la polyvinylpyrrolidone ;
3 - au moins un plastifiant présent à raison de 2 à 20 %, de préférence de 4 à 15 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine;
4 - et éventuellement au moins un agent tensioactif et/ou lubrifiant, présent à raison de 2 à 20 %, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et/ou oléique étant préférés, et/ou parmi les tensioactifs non ioniques, à savoir les esters de sorbitan polyoxyéthylénés et/ou les dérivés de l'huile de ricin polyoxyéthylénés, et/ou parmi les agents lubrifiants comme les stéarates de calcium, de magnésium, d'aluminium ou de zinc, ou comme le stéarylfumarate de sodium et/ou le béhénate de glycérol; ledit agent pouvant comprendre un seul ou un mélange des susdits produits ;
■ **en ce que** ces microcapsules possèdent une granulométrie comprise entre 50 et 1000 microns, de préférence entre 100 et 750 microns, et, plus préférentiellement encore, entre 200 et 500 microns.

2. Médicament selon la revendication 1, **caractérisé en ce que** la fraction massique moyenne en anti-hyperglycémiant des microcapsules est supérieure à 50%, de préférence supérieure ou égale à 60%.

3. Médicament selon la revendication 1 ou 2, **caractérisé en ce que**, dans un test de dissolution in vitro dénommé dissolutest de type II conformément à la Pharmacopée, la dissolution de (des) l'anti-hyperglycémiant(s) s'étend sur au moins 8 heures, de préférence au moins 20 heures.

4. Médicament selon l'une quelconque des revendications 1 à 3, **caractérisé en ce qu'**il se présente sous la forme d'un comprimé délitable dans la bouche ou par effervescence ou non dans un liquide, d'une poudre, d'une suspension où d'une gélule.

5. Médicament selon l'une quelconque des revendications 1 à 4, **caractérisé en ce qu'**il est constitué par une forme pharmaceutique dont la dose d'anti-hyperglycémiant(s) est comprise entre 800 et 1200 mg, de préférence entre 900 et 1100 mg, et plus préférentiellement encore de l'ordre de 1000 mg.

6. Médicament selon l'une quelconque des revendications 1 à 5, **caractérisé en ce que** ce médicament multi-microparticulaire se compose de plusieurs milliers de microcapsules telles que définies dans les revendications précédentes, cette multiplicité assurant une bonne reproductabilité du transit gastro-intestinal de (des) l'anti-hyperglycémiant(s), réduisant ainsi le risque d'hypoglycémie ou d'hyperglycémie pour le patient.

7. Médicament selon l'une quelconque des revendications 1 à 6, **caractérisé en ce que** le coeur des microcapsules est un granulé comportant de (des) l'anti-hyperglycémiant(s) et des excipients de granulation et/ou une particule d'anti-hyperglycémiant(s) sensiblement pur(s), de préférence un monocristal d'anti-hyperglycémiant(s).

8. Médicament selon l'une quelconque des revendications 1 à 7, **caractérisé en ce que** la pellicule d'enrobage comporte un ou plusieurs produits sélectionnés dans le groupe comprenant :
- les macromolécules filmogènes, de préférence choisies dans le groupe comprenant : éthers de cellulose, éthers / esters de cellulose, esters de cellulose, diesters de cellulose, triesters de cellulose, acylate de cellulose, diacylate de cellulose, triacylate de cellulose, diacétate et triacétate de cellulose, acétate propionate de cellulose, acétate butyrate de cellulose, poty-méthacrylates, cires, copolymères d'acétate de vinyle; l'éthylcelullose, l'Eudragit® RS, l'Eudragit® RL, l'acétate de cellulose étant particulièrement préférés ;
- les plastifiants, de préférence choisis parmi la liste non exhaustive suivante : acétyl tributyl citrate, acétyl triéthyl citrate, glycérides acétylés, huile de ricin, dibutylphtalate, diéthylphtalate, diéthylsébacate, dibutylsébacate, diméthylphtalate, glycérol, glycérol monostéarate, glycéryl triacétate, polyéthylène glycol, copolymères polyoxyéthylène-polyoxypropylène, propylène glycol, tributyl citrate, triéthylcitrate, esters d'acide citrique, adipate, azélate, benzoate, huiles végétales, sorbitol, diéthyloxalate, diéthylmalate, diéthylfumarate, dibutylsuccinate, diéthylmalonate, dioctylphtalate, glycéroltributyrate ;
- et éventuellement d'autres excipients sélectionnés parmi les charges solubles ou insolubles (talc, sels minéraux, sucres, polyvinylpyrrolidone, polyethylène glycol...), les lubrifiants, les colorants ou les pigments.

9. Médicament selon la revendication 8, **caractérisé en ce que** les macromolécules filmogènes constituant le pelliculage sont des associations de dérivés cellulosiques et d'au moins un polymère hydrophile pharmaceutiquement acceptable, la fraction massique en dérivés cellulosiques étant préférentiellement comprise entre 30 et 90%, et plus préférentiellement encore entre 50 et 80%.

10. Procédé de préparation du médicament tel que défini à l'une quelconque des revendications 1 à 9, **caractérisé en ce qu'**il consiste :
■ à mettre en oeuvre des granulés comportant de (des) l'anti-hyperglycémiant(s) et des X excipients de granulation ou bien de particules d'anti-hyperglycémiant(s) sensiblement pur(s), de préférence des monocristaux d'anti-hyperglycémiant(s) ;
■ puis à pulvériser sur ces granulés et/ou ces particules, une solution d'enrobage, comprenant un ou plusieurs produits sélectionnés dans le groupe comprenant :
o les macromolécules filmogènes, de préférence choisies dans le groupe comprenant : éthers de cellulose, éthers / esters de cellulose, esters de cellulose, diesters de cellulose, triesters de cellulose, acylate de cellulose, diacylate de cellulose, triacylate de cellulose, diacétate et triacétate de cellulose, acétate propionate de cellulose, acétate butyrate de cellulose, poly-méthacrylates, cires, copolymères d'acétate de vinyle ;
l'éthylcellulose, l'Eudragit® RS, l'Eudragit® RL, l'acétate de cellulose étant particulièrement préférés ;
o les plastifiants, de préférence choisis parmi la liste non exhaustive suivante : acétyl tributyl citrate, acétyle triéthyl citrate, glycérides acétylés, huile de ricin, dibutylphtalate, diéthylphtalate, diéthylsébacate, dibutylsébacate, diméthylphtalate, glycérol, glycérol monostéarate, glycéryl triacétate, polyéthylène glycol, copolymères polyoxyéthylène / polyoxypropylène, propylène glycol, tributyl citrate, triéthylcitrate, esters d'acide citrique, adipate, azélate, benzoate, huiles végétales, sorbitol, diéthyloxalate, diéthylmalate, diéthylfumarate, dibutylsuccinate, diéthylmalonate, dioctylphtalate, glycéroltributyrate,
o et éventuellement d'autres excipients sélectionnés parmi les charges solubles ou insolubles (talc, sels minéraux, sucres, polyvinylpyrrolidone, polyéthylène glycol...), les lubrifiants, les colorants et les pigments.

11. Utilisation d'une pluralité de microcapsules
■ constituées chacune par un coeur comportant au moins un anti-hyperglycémiant choisi parmi les biguanides, de préférence dans le groupe de biguanides comprenant la metformine et la buformine et leurs sels, la metformine et ses sels étant particulièrement préférés, et par une pellicule d'enrobage appliquée sur le coeur et permettant la libération prolongée in vivo de (des) l'anti-hyperglycémiant(s), à l'exclusion des pellicules d'enrobage constituées par des compositions entériques et des pellicules d'enrobage de composition ci-après :
1 - au moins un polymère filmogène (P1) insoluble dans les liquides du tractus, présent à raison de 50 à 90 %, de préférence 50 à 80 % en poids sur sec par rapport à la masse totale de la composition d'enrobage et constitué par au moins un dérivé non hydrosoluble de la cellulose, à savoir l'éthylcellulose et/ou l'acétate de cellulose ;
2 - au moins un polymère azoté (P2) présent à raison de 2 à 25 %, de préférence 5 à 15 % en poids sur sec par rapport à la masse totale de la composition d'enrobage et constitué par au moins un polyacrylamide et/ou un poly-N-vinylamide et/ou un poly-N-vinyl-lactame, à savoir le polyacrylamide et/ou la polyvinylpyrrolidone ;
3 - au moins un plastifiant présent à raison de 2 à 20 %, de préférence de 4 à 15 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine ;
4 - et éventuellement au moins un agent tensioactif et/ou lubrifiant, présent à raison de 2 à 20 %, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et/ou oléique étant préférés, et/ou parmi les tensioactifs non ioniques, à savoir les esters de sorbitan polyoxyéthylénés et/ou les dérivés de l'huile de ricin polyoxyéthylénés, et/ou parmi les agents lubrifiants comme les stéarates de calcium, de magnésium, d'aluminium ou de zinc, ou comme le stéarylfumarate de sodium et/ou le béhénate de glycérol; ledit agent pouvant comprendre un seul ou un mélange des susdits produits ;
■ et possédant une granulométrie comprise entre 50 et 1 000 microns, de préférence entre 100 et 750 microns, et, plus préférentiellement encore, entre 200 et 500 microns ;
pour fabriquer un médicament à base d'au moins un anti-hyperglycémiant, administrable par voie orale, avalable aisément et qui contribue au contrôle de la glycémie sur au moins 12h, de manière sûre, et en limitant les problèmes de bipasse, d'accumulation et de libération localisée et massive d'anti-hyperglycémiant.

## Claims

1. A medicament based on at least one antihyperglycemic which can be administered by the oral route, chosen from the biguanides, preferably from the group of biguanides comprising metformin and buformin and their salts, metformin and its salts being particularly preferred,
**characterized:**
■ **in that** it comprises a plurality of micro-capsules each composed of a core comprising at least one antihyperglycemic and of a coating film applied to the core and which makes possible the prolonged release in vivo of the antihyperglycemic(s),
with the exclusion of the coating films composed of enteric compositions and of the coating films with the following composition:
1 - at least one film-forming polymer (P1) which is insoluble in the liquids of the tract, present in a proportion of 50 to 90%, preferably 50 to 80%, by weight on a dry basis with respect to the total mass of the coating composition and composed of at least one water-insoluble derivative of cellulose, namely ethylcellulose and/or cellulose acetate;
2 - at least one nitrogenous polymer (P2), present in a proportion of 2 to 25%, preferably 5 to 15%, by weight on a dry basis with respect to the total mass of the coating composition and composed of at least one polyacrylamide and/or one poly-N-vinylamide and/or one poly-N-vinyllactam, namely polyacrylamide and/or polyvinylpyrrolidone;
3 - at least one plasticizer, present in a proportion of 2 to 20%, preferably of 4 to 15%, by weight on a dry basis with respect to the total mass of the coating composition and composed of at least one of the following compounds: glycerol esters, phthalates, citrates, sebacates, esters of cetyl alcohol, castor oil, salicylic acid and cutin;
4 - and optionally at least one surface-active and/or lubricating agent, present in a proportion of 2 to 20%, preferably of 4 to 15%, by weight on a dry basis with respect to the total mass of the coating composition and chosen from anionic surfactants, namely alkali metal or alkaline earth metal salts of fatty acids, stearic acid and/or oleic acid being preferred, and/or from nonionic surfactants, namely polyoxyethylenated sorbitan esters and/or polyoxyethylenated castor oil derivatives, and/or from lubricating agents, such as calcium stearate, magnesium stearate, aluminum stearate or zinc stearate, or such as sodium stearylfumarate and/or glycerol behenate; it being possible for said agent to comprise just one or a mixture of abovesaid products;
■ **in that** these microcapsules have a particle size of between 50 and 1000 microns, preferably between 100 and 750 microns and more preferably still between 200 and 500 microns.

2. The medicament as claimed in claim 1, **characterized in that** the mean fraction by mass of antihyperglycemic in the microcapsules is greater than 50%, preferably greater than or equal to 60%.

3. The medicament as claimed in claim 1 or 2, **characterized in that**, in an in vitro dissolution test known as the type II dissolutest in accordance with the Pharmacopoeia, the dissolution of the antihyperglycemic(s) extends over at least 8 hours, preferably at least 20 hours.

4. The medicament as claimed in any one of claims 1 to 3, **characterized in that** it exists in the form of a tablet which can disintegrate in the mouth or, by effervescence or not by effervescence, in a liquid, of a powder, of a suspension or of a gelatin capsule.

5. The medicament as claimed in any one of claims 1 to 4, **characterized in that** it is composed of a pharmaceutical dosage form, the dose of antihyperglycemic(s) of which is between 800 and 1200 mg, preferably between 900 and 1100 mg and more preferably still of the order of 1000 mg.

6. The medicament as claimed in any one of claims 1 to 5, **characterized in that** this multimicrocapsule medicament is composed of several thousand microcapsules as defined in the preceding claims, this multiplicity providing good reproducibility of the gastrointestinal transit of the anti-hyperglycemic(s), thus reducing the risk to the patient of hypoglycemia or hyperglycemia.

7. The medicament as claimed in any one of claims 1 to 6, **characterized in that** the core of the micro-capsules is a granule comprising anti-hyperglycemic(s) and granulation excipients and/or a particle of substantially pure antihyperglycemic(s), preferably a monocrystal of antihyperglycemic(s).

8. The medicament as claimed in any one of claims 1 to 7, **characterized in that** the coating film comprises one or more products selected from the group consisting of:
- film-forming macromolecules, preferably chosen from the group consisting of: cellulose ethers, cellulose ethers/esters, cellulose esters, cellulose diesters, cellulose triesters, cellulose acylate, cellulose diacylate, cellulose triacylate, cellulose diacetate and triacetate, cellulose acetate/propionate, cellulose acetate/butyrate, polymethacrylates, waxes and vinyl acetate copolymers;
ethylcellulose, Eudragit® RS, Eudragit® RL and cellulose acetate being particularly preferred;
- plasticizers, preferably chosen from the following nonexhaustive list: tributyl acetylcitrate, triethyl acetylcitrate, acetylated glycerides, castor oil, dibutyl phthalate, diethyl phthalate, diethyl sebacate, dibutyl sebacate, dimethyl phthalate, glycerol, glycerol monostearate, glyceryl triacetate, polyethylene glycol, polyoxyethylene/polyoxypropylene copolymers, propylene glycol, tributyl citrate, triethyl citrate, citric acid esters, adipate, azelate, benzoate, vegetable oils, sorbitol, diethyl oxalate, diethyl malate, diethyl fumarate, dibutyl succinate, diethyl malonate, dioctyl phthalate or glycerol tributyrate;
- and optionally other excipients selected from soluble or insoluble fillers (talc, inorganic salts, sugars, polyvinylpyrrolidone, polyethylene glycol, and the like), lubricants, dyes or pigments.

9. The medicament as claimed in claim 8, **characterized in that** the film-forming macromolecules constituting the film coating are combinations of cellulose derivatives and of at least one pharmaceutically acceptable hydrophilic polymer, the fraction by mass of cellulose derivatives preferably being between 30 and 90% and more preferably still between 50 and 80%.

10. A process for the preparation of the medicament as claimed in any one of claims 1 to 9, **characterized in that** it consists:
■ in employing granules comprising antihyperglycemic(s) and granulation excipients or alternatively particles of substantially pure antihyperglycemic(s), preferably monocrystals of antihyperglycemic(s);
■ and in then spraying, over these granules and/or these particles, a coating solution comprising one or more products selected from the group consisting of:
o film-forming macromolecules, preferably chosen from the group consisting of: cellulose ethers, cellulose ethers/esters, cellulose esters, cellulose diesters, cellulose triesters, cellulose acylate, cellulose diacylate, cellulose triacylate, cellulose diacetate and triacetate, cellulose acetate/propionate, cellulose acetate/butyrate, polymethacrylates, waxes and vinyl acetate copolymers;
ethylcellulose, Eudragit® RS, Eudragit® RL and cellulose acetate being particularly preferred;
o plasticizers, preferably chosen from the following nonexhaustive list: tributyl acetylcitrate, triethyl acetylcitrate, acetylated glycerides, castor oil, dibutyl phthalate, diethyl phthalate, diethyl sebacate, dibutyl sebacate, dimethyl phthalate, glycerol, glycerol monostearate, glyceryl triacetate, polyethylene glycol, polyoxyethylene/polyoxypropylene copolymers, propylene glycol, tributyl citrate, triethyl citrate, citric acid esters, adipate, azelate, benzoate, vegetable oils, sorbitol, diethyl oxalate, diethyl malate, diethyl fumarate, dibutyl succinate, diethyl malonate, dioctyl phthalate or glycerol tributyrate;
o and optionally other excipients selected from soluble or insoluble fillers (talc, inorganic salts, sugars, polyvinylpyrrolidone, polyethylene glycol, and the like), lubricants, dyes or pigments.

11. Use of a plurality of microcapsules:
■ each composed of a core comprising at least one antihyperglycemic chosen from the biguanides, preferably from the group of biguanides comprising metformin and buformin and their salts, metformin and its salts being particularly preferred, and of a coating film applied to the core and which makes possible the prolonged release in vivo of the anti-hyperglycemic(s),
with the exclusion of the coating films composed of enteric compositions and of the coating films with the following composition:
1- at least one film-forming polymer (P1) which is insoluble in the fluids of the tract, present in a proportion of 50 to 90%, preferably 50 to 80%, by weight on a dry basis with respect to the total mass of the coating composition and composed of at least one water-insoluble cellulose derivative of cellulose, namely ethylcellulose and/or cellulose acetate;
2- at least one nitrogenous polymer (P2), present in a proportion of 2 to 25%, preferably 5 to 15%, by weight on a dry basis with respect to the total mass of the coating composition and composed of at least one polyacrylamide and/or one poly-N-vinylamide and/or one poly-N-vinyllactam, namely polyacrylamide and/or polyvinylpyrrolidone;
3- at least one plasticizer, present in a proportion of 2 to 20%, preferably of 4 to 15%, by weight on a dry basis with respect to the total mass of the coating composition and composed of at least one of the following compounds: glycerol esters, phthalates, citrates, sebacates, esters of cetyl alcohol, castor oil, salicylic acid and cutin;
4- and optionally at least one surface-active and/or lubricating agent, present in a proportion of 2 to 20%, preferably of 4 to 15%, by weight on a dry basis with respect to the total mass of the coating composition and chosen from anionic surfactants, namely alkali metal or alkaline earth metal salts of fatty acids, stearic acid and/or oleic acid being preferred, and/or from nonionic surfactants, namely polyoxyethylenated sorbitan esters and/or polyoxyethylenated castor oil derivatives, and/or from lubricating agents, such as calcium stearate, magnesium stearate, aluminum stearate or zinc stearate, or such as sodium stearylfumarate and/or glycerol behenate; it being possible for said agent to comprise just one or a mixture of abovesaid products;
■ and having a particle size of between 50 and 1000 microns, preferably between 100 and 750 microns and more preferably still between 200 and 500 microns;
for manufacturing a medicament based on at least one antihyperglycemic which can be administered by the oral route, which can be easily swallowed and which contributes to the control of glycemia over at least 12 h in a reliable manner and while limiting problems of bypass, of accumulation and of localized and massive release of antihyperglycemic.

## Patentansprüche

1. Medikament auf der Basis wenigstens eines antihyperglykämischen Wirkstoffs zur oralen Verabreichung, der aus Biguaniden, vorzugsweise aus der Gruppe der Biguanide, die Metformin und Buformin und ihre Salze umfassen, ausgewählt ist, wobei Metformin und seine Salze besonders bevorzugt sind;
**dadurch gekennzeichnet:**
■ **dass** es eine Vielzahl von Mikrokapseln umfasst, die jeweils aus einem Kern, der wenigstens einen antihyperglykämischen Wirkstoff umfasst, und einem Beschichtungsfilm, der auf den Kern aufgetragen wird und die verlängerte Freisetzung des (bzw. der) antihyperglykämischen Wirkstoffs(e) in vivo erlaubt, besteht;
mit Ausnahme der Beschichtungsfilme, die aus magensaftresistenten Zusammensetzungen bestehen, und der Beschichtungsfilme mit der folgenden Zusammensetzung:
1 - wenigstens ein filmbildendes Polymer (P1), das in den Flüssigkeiten des Magen-Darm-Trakts unlöslich ist und in einem Anteil von 50 bis 90 Gew.-%, vorzugsweise 50 bis 80 Gew.-%, Trockengewicht in Bezug auf die Gesamtmasse der Beschichtungszusammensetzung vorhanden ist und aus wenigstens einem wasserunlöslichen Cellulosederivat, nämlich Ethylcellulose und/oder Celluloseacetat besteht;
2 - wenigstens ein stickstoffhaltiges Polymer (P2), das in einem Anteil von 2 bis 25 Gew.-%, vorzugsweise 5 bis 15 Gew.-%, Trockengewicht in Bezug auf die Gesamtmasse der Beschichtungszusammensetzung vorhanden ist und aus wenigstens einem Polyacrylamid und/oder Poly-N-vinylamid und/oder Poly-N-vinyllactam, nämlich Polyacrylamid und/oder Polyvinylpyrrolidon, besteht;
3 - wenigstens ein Weichmacher, der in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus wenigstens einer der folgenden Verbindungen besteht: Glycerinestern, Phthalaten, Citraten, Sebacaten, Estern von Cetylalkohol, Ricinusöl, Salicylsäure und Cutin;
4 - und gegebenenfalls wenigstens ein Tensid und/oder Gleitmittel, das in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus anionischen Tensiden, nämlich Alkalimetall- oder Erdalkalimetallsalzen von Fettsäuren, wobei Stearinsäure und/oder Ölsäure bevorzugt sind, und/oder nichtionischen Tensiden, nämlich polyoxyethylenierten sorbitan-estern und/oder polyoxyethylierten Derivaten von Ricinusöl, und/oder Gleitmitteln, wie Calcium-, Magnesium-, Aluminium- oder Zinkstearaten oder Natriumstearylfumarat und/oder Glycerinbehenat, ausgewählt ist, wobei das Tensid bzw. Gleitmittel ein einziges oder ein Gemisch der obigen Produkte umfassen kann;
■ **dass** diese Mikrokapseln eine Teilchengröße zwischen 50 und 1000 µm, vorzugsweise zwischen 100 und 750 µm und besonders bevorzugt zwischen 200 und 500 µm haben.

2. Medikament gemäß Anspruch 1, **dadurch gekennzeichnet, dass** der mittlere Massenanteil an antihyperglykämischem Wirkstoff in den Mikrokapseln über 50% liegt, vorzugsweise größer oder gleich 60% ist.

3. Medikament gemäß Anspruch 1 oder 2, **dadurch gekennzeichnet, dass** sich in einem in-vitro-Auflösungstest, der als Auflösungstest des Typs II gemäß Arzneibuch bezeichnet wird, die Auflösung des (bzw. der) antihyperglykämischen Wirkstoffs(e) über wenigstens 8 Stunden, vorzugsweise wenigstens 20 Stunden, erstreckt.

4. Medikament gemäß einem der Ansprüche 1 bis 3, **dadurch gekennzeichnet, dass** es in Form einer Tablette, die im Mund zerfällt oder die im Flüssigkeit mit Sprudeln oder nicht zerfällt, eines Pulvers, einer Suspension oder eines Gels vorliegt.

5. Medikament gemäß einem der Ansprüche 1 bis 4, **dadurch gekennzeichnet, dass** es aus einer pharmazeutischen Form besteht, deren Dosis des (bzw. der) antihyperglykämischen Wirkstoffs(e) zwischen 800 und 1200 mg, vorzugsweise zwischen 900 und 1100 mg und besonders bevorzugt in der Größenordnung von 1000 mg liegt.

6. Medikament gemäß einem der Ansprüche 1 bis 5, **dadurch gekennzeichnet, dass** dieses Medikament multimikropartikulär ist und aus mehreren Tausend Mikrokapseln besteht, wie die in den vorstehenden Ansprüchen definiert sind, wobei diese Vielfalt eine gute Reproduzierbarkeit der Magen-Darm-Passage des (bzw. der) antihyperglykämischen Wirkstoffs(e) gewährleistet und somit das Risiko einer Hypoglykämie oder Hyperglykämie für den Patienten reduziert.

7. Medikament gemäß einem der Ansprüche 1 bis 6, **dadurch gekennzeichnet, dass** der Kern der Mikrokapseln ein Korn ist, das einen (bzw. mehrere) antihyperglykämische Wirkstoffs(e) und Granulierungshilfsstoffe und/oder ein oder mehrere im Wesentlichen reine antihyperglykämische Teilchen, vorzugsweise einen antihyperglykämischen Einkristall, umfasst.

8. Medikament gemäß einem der Ansprüche 1 bis 7, **dadurch gekennzeichnet, dass** der Beschichtungsfilm ein oder mehrere Produkte umfasst, die aus der Gruppe ausgewählt sind, die Folgendes umfasst:
- filmbildende Makromoleküle, die vorzugsweise aus der Gruppe ausgewählt sind, die Folgendes umfasst: Celluloseethern, Celluloseethern/estern, Celluloseestern, Cellulosediestern, Cellulosetriestern, Celluloseacylat, Cellulosediacylat, Cellulosetriacylat, Cellulosediacetat und -triacetat, Celluloseacetatpropionat, Celluloseacetatbutyrat, Polymethacrylaten, Wachse, Vinylacetatcopolymeren, wobei Ethylcellulose, Eudragit^{®} RS, Eudragit^{®} RL und Celluloseacetat besonders bevorzugt sind;
- Weichmachern, die vorzugsweise aus der folgenden nichtumfassenden Liste ausgewählt sind: Acetyltributylcitrat, Acetyltriethylcitrat, acetylierte Glyceride, Ricinusöl, Dibutylphthalat, Diethylphthalat, Diethylsebacat, Dibutylsebacat, Dimethylphthalat, Glycerin, Glycerinmonostearat, Glycerintriacetat, Polyethylenglycol, Polyoxyethylen-/Polyoxypropylen-Copolymere, Propylenglycol, Tributylcitrat, Triethylcitrat, Zitronensäureester, Adipat, Azelat, Benzoat, Pflanzenöle, Sorbit, Diethyloxalat, Diethylmalat, Diethylfumarat, Dibutylsuccinat, Diethylmalonat, Dioctylphthalat, Glycerintributyrat;
- und gegebenenfalls andere Arzneimittelhilfsstoffe, die aus löslichen oder unlöslichen Füllstoffen (Talk, Mineralsalzen, Zuckern, Polyvinylpyrrolidon, Polyethylenglycol usw.), Gleitmitteln, Farbstoffen oder Pigmenten ausgewählt sind.

9. Medikament gemäß Anspruch 8, **dadurch gekennzeichnet, dass** die filmbildenden Makromoleküle, die die Beschichtung bilden, Kombinationen von Cellulosederivaten und wenigstens einem pharmazeutisch annehmbaren hydrophilen Polymer sind, wobei der Massenanteil der Cellulosederivate vorzugsweise zwischen 30 und 90% und besonders bevorzugt zwischen 50 und 80% liegt.

10. Verfahren zur Herstellung des Medikaments, wie es in einem der Ansprüche 1 bis 9 definiert ist, **dadurch gekennzeichnet, dass** es die folgenden Schritte umfasst:
■ Bereitstellen von Körnern, die einen (bzw. mehrere) antihyperglykämische Wirkstoffs(e) und Granulierungshilfsstoffe umfassen, oder auch von einem oder mehreren im Wesentlichen reinen antihyperglykämischen Teilchen, vorzugsweise einem oder mehreren antihyperglykämischen Einkristallen;
■ danach Aufsprühen einer Beschichtungslösung auf diese Körner und/oder Teilchen, wobei die Beschichtungslösung ein oder mehrere Produkte umfasst, die aus der Gruppe ausgewählt sind, die Folgendes umfasst:
o Filmbildende Makromoleküle, die vorzugsweise aus der Gruppe ausgewählt sind, die Folgendes umfasst: Celluloseethern, Celluloseethern/estern, Celluloseestern, Cellulosediestern, Cellulosetriestern, Celluloseacylat, Cellulosediacylat, Cellulosetriacylat, Cellulosediacetat und -triacetat, Celluloseacetatpropionat, Celluloseacetatbutyrat, Polymethacrylaten, Wachse, Vinylacetatcopolymeren, wobei Ethylcellulose, Eudragit^{®} RS, Eudragit^{®} RL und Celluloseacetat besonders bevorzugt sind;
o Weichmachern, die vorzugsweise aus der folgenden nichtumfassenden Liste ausgewählt sind: Acetyltributylcitrat, Acetyltriethylcitrat, acetylierte Glyceride, Ricinusöl, Dibutylphthalat, Diethylphthalat, Diethylsebacat, Dibutylsebacat, Dimethylphthalat, Glycerin, Glycerinmonostearat, Glycerintriacetat, Polyethylenglycol, Polyoxyethylen-/Polyoxypropylen-Copolymere, Propylenglycol, Tributylcitrat, Triethylcitrat, Zitronensäureester, Adipat, Azelat, Benzoat, Pflanzenöle, Sorbit, Diethyloxalat, Diethylmalat, Diethylfumarat, Dibutylsuccinat, Diethylmalonat, Dioctylphthalat, Glycerintributyrat;
o und gegebenenfalls andere Arzneimittelhilfsstoffe, die aus löslichen oder unlöslichen Füllstoffen (Talk, Mineralsalzen, Zuckern, Polyvinylpyrrolidon, Polyethylenglycol usw.), Gleitmitteln, Farbstoffen oder Pigmenten ausgewählt sind.

11. Verwendung einer Vielzahl von Mikrokapseln,
■ die jeweils aus einem Kern, der wenigstens einen antihyperglykämischen Wirkstoff umfasst, der aus Biguaniden, vorzugsweise aus der Gruppe der Biguanide, die Metformin und Buformin und ihre Salze umfassen, ausgewählt ist, wobei Metformin und seine Salze besonders bevorzugt sind, und einem Beschichtungsfilm, der auf den Kern aufgetragen wird und die verlängerte Freisetzung des (bzw. der) antihyperglykämischen Wirkstoffs(e) in vivo erlaubt, besteht;
mit Ausnahme der Beschichtungsfilme, die aus magensaftresistenten Zusammensetzungen bestehen, und der Beschichtungsfilme mit der folgenden Zusammensetzung:
1 - wenigstens ein filmbildendes Polymer (P1), das in den Flüssigkeiten des Magen-Darm-Trakts unlöslich ist und in einem Anteil von 50 bis 90 Gew.-%, vorzugsweise 50 bis 80 Gew.-%, Trockengewicht in Bezug auf die Gesamtmasse der Beschichtungszusammensetzung vorhanden ist und aus wenigstens einem wasserunlöslichen Cellulosederivat, nämlich Ethylcellulose und/oder Celluloseacetat besteht;
2 - wenigstens ein stickstoffhaltiges Polymer (P2), das in einem Anteil von 2 bis 25 Gew.-%, vorzugsweise 5 bis 15 Gew.-%, Trockengewicht in Bezug auf die Gesamtmasse der Beschichtungszusammensetzung vorhanden ist und aus wenigstens einem Polyacrylamid und/oder Poly-N-vinylamid und/oder Poly-N-vinyllactam, nämlich Polyacrylamid und/oder Polyvinylpyrrolidon, besteht;
3 - wenigstens ein Weichmacher, der in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus wenigstens einer der folgenden Verbindungen besteht: Glycerinestern, Phthalaten, Citraten, Sebacaten, Estern von Cetylalkohol, Ricinusöl, Salicylsäure und Cutin;
4 - und gegebenenfalls wenigstens ein Tensid und/oder Gleitmittel, das in einem Anteil von 2 bis 20 Gew.-%, vorzugsweise 4 bis 15 Gew.-%, Trockengewicht in Bezug auf die Gesamtmasse der Beschichtungszusammensetzung vorhanden ist und aus anionischen Tensiden, nämlich Alkalimetall- oder Erdalkalimetallsalzen von Fettsäuren, wobei Stearinsäure und/oder Ölsäure bevorzugt sind, und/oder nichtionischen Tensiden, nämlich Polyoxyethylenierten sorbitanestern und/oder polyoxyethylierten Derivaten von Ricinusöl, und/oder Gleitmitteln, wie Calcium-, Magnesium-, Aluminium- oder Zinkstearaten oder Natriumstearylfumarat und/oder Glycerinbehenat, ausgewählt ist, wobei das Tensid bzw. Gleitmittel ein einziges oder ein Gemisch der obigen Produkte umfassen kann;
■ und die eine Teilchengröße zwischen 50 und 1000 µm, vorzugsweise zwischen 100 und 750 µm und besonders bevorzugt zwischen 200 und 500 µm haben;
zur Herstellung eines Medikaments auf der Basis wenigstens eines antihyperglykämischen Wirkstoffs zur oralen Verabreichung, das leicht zu schlucken ist und während wenigstens 12 h zur sicheren Steuerung der Glykämie beiträgt und die Probleme der Umleitung, Anhäufung und massiven lokalisierten Freisetzung des antihyperglykämischen Wirkstoffs einschränkt.
